(19) Europäisches Patentamt
European Patent Office
Office européen des brevets

(11) Publication number: **0 181 592 B1**

(12) **EUROPEAN PATENT SPECIFICATION**

(45) Date of publication of patent specification: **16.09.92**

(51) Int. Cl.⁵: **C07C 279/04**, C07C 279/18, A61K 31/16

(21) Application number: **85114042.6**

(22) Date of filing: **05.11.85**

# (54) Use of spergualin derivatives for the preparation of medicaments having immunosuppressive activity.

(30) Priority: **13.11.84 JP 237480/84**

(43) Date of publication of application:
**21.05.86 Bulletin 86/21**

(45) Publication of the grant of the patent:
**16.09.92 Bulletin 92/38**

(84) Designated Contracting States:
**AT BE CH DE FR GB IT LI LU NL SE**

(56) References cited:
**EP-A- 0 094 632**
**EP-A- 0 105 193**
**EP-A- 0 153 720**
**EP-A- 0 188 821**
**EP-A- 0 213 526**
**FR-A- 2 514 350**
**GB-A- 2 155 013**

**Mutschler, E., "Arzneimittelmischungen", 4th ed., 1981, p. 583**

**Kirk-Othmer, "Encyclopaedia of Chemical Technology", vol. 13, 1981, p. 176**

**THE MERCK MANUAL, 14th ed. 1982, pages 342-247, R. BERKOW M.D., Merck & Co., Inc., Rahway, N.J., USA**

(73) Proprietor: **MICROBIAL CHEMISTRY RESEARCH FOUNDATION**
**14-23, Kamiosaki 3 Chome Shinagawa-ku**
**Tokyo 141(JP)**

(72) Inventor: **Umezawa, Hamao, Prof.**
**23 Toyotama-kita 4-chome**
**Nerima-ku Tokyo(JP)**
Inventor: **Takeuchi, Tomio**
**1-11, Higashigotanda 5-chome**
**Shinagawa-ku Tokyo(JP)**
Inventor: **Ishizuka, Masaaki**
**17, Denenchofu-honcho 3 chome**
**Ohta-ku Tokyo(JP)**
Inventor: **Abe, Fuminori**
**29-15, Shimo 3-chome**
**Kita-ku Tokyo(JP)**
Inventor: **Fujii, Akio**
**8-13, Ofuna 4-chome**
**Kamakura-city Kanagawa Prefecture(JP)**
Inventor: **Nakamura, Teruya**
**831, Nomura-cho**
**Kusatsu-city Shiga Prefecture(JP)**

**EP 0 181 592 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**THE JOURNAL OF ANTIBIOTICS, vol. 38, no. 2, February 1985, pages 283-4; H. UMEZAWA et al, "Suppression of tissue graft rejection by spergualin"**

**THE JOURNAL OF ANITBIOTICS, vol. 39, no. 10, October 1986, pages 1461-66; K. NISHIKAWA et al, "Antitumor activity of spergualin, a novel antitumor antibiotic"**

(74) Representative: **Becker, Heinrich Karl Engelbert, Dr. et al**
**HOECHST AKTIENGESELLSCHAFT Central Patent Department P.O. Box 80 03 20**
**W-6230 Frankfurt am Main 80(DE)**

## Description

The present invention relates to immunosuppressants containing Spergualin compounds as an active ingredient.

A number of drugs with immunosuppressive actions are already known. They include alkylating agents, antimetabolites, antibiotics, steroids, folic acid antagonists and plant alkaloids.

Spergualin is a compound that was isolated by Umezawa and others from the filtrate of a culture broth of Spergualin-producing microorganism of the genus Bacillus. The structural formula of Spergualin is shown below:

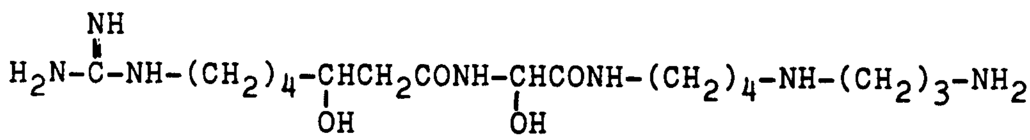

Spergualin is not only effective against mouse leukemia L-1210, mouse leukemia EL-4 , Ehrlich carcinoma and sarcoma 180 bus also exhibits promising activity in controlling malignant tumors (see Japanese Unexamined Published Patent Application No. 48957/1982).

Umezawa et al. continued their studies on Spergualin compounds and have synthesized numerous Spergualin compounds which are more stable and have a stronger anti-tumor activity (see Japanese Unexamined Published Patent Application Nos. 62152/1983 ( = FR-A-2514350),42356/1984 ( = EP-A-105193) and 76046/1984 ( = JP 59-76046 ) and European Patent Application Nos. 94632 and 153720. Among these compounds are those used in the present invention.

The immunosuppressive effects of steroids are considered to be accomplished principally through the anti-inflammatory action and the lysis of lymphocytes. However, as is well known, steroids have diversified pharmacological effects and cause many side effects. The other immunosuppressants are classified as "cytotoxic" substances and their action is directed, among other things, to the pathways of nucleic acid synthesis and may often cause serious side effects on hematopoietic organs. It is therefore desired to develop immunosuppressive drugs that act selectively on lymphocytes and other cells of immunological importance while causing minimum side effects.

The present invention relates to the use of Spergualin compounds of formula (I) shown below:

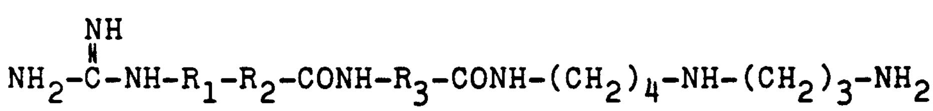

(I)

wherein $R_1$ is $-(CH_2)_4-$, $-(CH_2)_6-$,

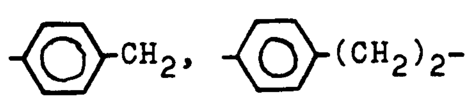

or

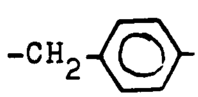

;

$R_2$ is $-(CH_2)_2-$ or $-CH=CH-$; and $R_3$ is

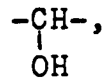

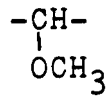

or $-CH_2-$or pharmacologically acceptable salts thereof, for the preparation of medicaments having immunosuppressive activity.

Preferred compounds are those wherein $R_1$ is $-(CH_2)_4-$or $-(CH_2)_6-$; $R_2$ is $-(CH_2)_2-$ or -CH = CH-(trans); and $R_3$ is

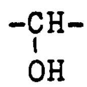, 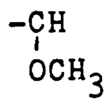

or $-CH_2-$; or pharmacologically acceptable salts thereof.

Specific examples of the compounds usable as immunosuppressive agents in accordance with the present invention are listed below:

(1) N-[4-(3-aminopropyl)aminobutyl]-2-(7-guanidinoheptaneamido)-2-hydroxyethaneamide;
(2) N-[4-(3-aminopropyl)aminobutyl]-2-(7-guanidinoheptaneamino)-2-methoxyethaneamide;
(3) N-[4-(3-aminopropyl)aminobutyl]-2-(9-guanidinononaneamido)-2-hydroxyethaneamide;
(4) N-[4-(3-aminopropyl)aminobutyl]-2-(7-guanidinoheptaneamido)-ethaneamide;
(5) N-[4-(3-aminopropyl)aminobutyl]-2-(7-guanidinoheptaneamido)-(S)-2-hydroxymethylethaneamide;
(6) N-[4-(3-aminopropyl)aminobutyl]-2-[4-(p-guanidinophenyl)butaneamido]ethaneamide;
(7) N-[4-(3-aminopropyl)aminobutyl]-2-[4-(p-guanidinophenyl)-butaneamido]-(S)-2-hydroxymethylethaneamide;
(8) N-[4-(3-aminopropyl)aminobutyl]-2-[3-(p-guanidinomethylphenyl)propaneamido]-(S)-2-hydroxymethylethaneamide;
(9) N-[4-(3-aminopropyl)aminobutyl]-2-[5-(p-guanidinophenyl)-pentaneamido]-(S)-2-hydroxymethylethaneamide;
(10) N-[4-(3-aminopropyl)aminobutyl]-2-[7-guanidinohepta-2-enamido]-2-methoxyethaneamide; and
(11) N-[4-(3-aminopropyl)aminobutyl]-2-[7-guanidinohepta-2-enamido]-2-hydroxyethaneamide.

These compounds have the structures shown in Table 1.

**Table 1** Chemicals Structures of Typical Examples of the Compounds used for the Present Invention

$$NH_2-\overset{NH}{\overset{\|}{C}}-NH-R_1-R_2-CONH-R_3CO-NH-(CH_2)_4-NH-(CH_2)_3-NH_2$$

| Compound No. | $R_1$ | $R_2$ | $R_3$ |
|---|---|---|---|
| (1) | $-(CH_2)_4-$ | $-(CH_2)_2-$ | $-\underset{OH}{\underset{\mid}{CH}}-$ |
| (2) | $-(CH_2)_4-$ | $-(CH_2)_2-$ | $-\underset{OCH_3}{\underset{\mid}{CH}}-$ |

| Compound No. | $R_1$ | $R_2$ | $R_3$ |
|---|---|---|---|
| (3) | $-(CH_2)_6-$ | $-(CH_2)_2-$ | -CH-<br>OH |
| (4) | $-(CH_2)_4-$ | $-(CH_2)_2-$ | $-CH_2-$ |
| (5) | $-(CH_2)_4-$ | $-(CH_2)_2-$ | (S)<br>-CH-<br>$CH_2OH$ |
| (6) | 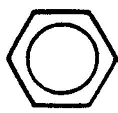 $-CH_2-$ | $-(CH_2)_2-$ | $-CH_2-$ |
| (7) | 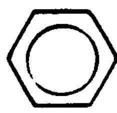 $-CH_2-$ | $-(CH_2)_2-$ | (S)<br>-CH-<br>$CH_2OH$ |
| (8) | $-CH_2-$ 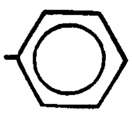 | $-(CH_2)_2-$ | (S)<br>-CH-<br>$CH_2OH$ |
| (9) | 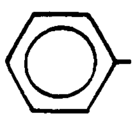 $-(CH_2)_2-$ | $-(CH_2)_2-$ | (S)<br>-CH-<br>$CH_2OH$ |
| (10) | $-(CH_2)_4-$ | -CH=CH-<br>(trans) | -CH-<br>OH |
| (11) | $-(CH_2)_6-$ | -CH=CH-<br>(trans) | -CH-<br>OH |

The compounds of formula (I) may form salts with acids. Salt-forming acids may be inorganic or organic if they are physiologically accpetable. Preferred inorganic acids are hydrochloric acid, sulfuric acid, nitric acid and phosphoric acid; preferred organic acids include acetic acid, propionic acid, succinic acid, fumaric acid, maleic acid, malic acid, tartaric acid, glutaric acid, citric acid, benzenesulfonic acid, toluensulfonic acid, toluenesulfonic acid, methanesulfonic acid, ethanesulfonic acid, propanesulfonic acid, aspartic acid and glutamic acid.

Among the compounds listed above, (1) to (3) are known and described in Japanese Unexamined Published Patnet Application No. 62152/1983 (= FR-A-2514350); compounds (4) and (5) are also known and described in Japanese Unexamined Published Patent Application No. 42356/1984 (= EP-A-105193); compounds (6) to (9) are also known and described in EP-A-153720; compounds (10 and (11) are also

known and described in Japanese Unexamined Published Patent Application No. 76046/1984 (= JP 59-76046). These known compounds may be synthesized by known methods.

When the compounds are used as immunosuppressants according to the present invention, they are administered either independently or in admixture with excipients or carriers to form injections, oral formulations or suppositories. Pharmaceutically acceptable excipients and carriers should be selected and their type and composition are determined by the route and method of administration. Illustrative liquid carriers include water, alcohols, as well as animal, vegetable and synthetic oils such as soybean oil, peanut oil, sesame oil and mineral oils. Exemplary solid carriers include sugars such as maltose and sucrose, amino acids, cellulose derivatives such as hydroxypropyl cellulose, and organic acid salts such as magnesium stearate. Carriers suitable for use in preparing injections include physiological saline, buffer solutions, solutions of sugars such as glucose, inositol, and mannitol, and glycols such as ethylene glycol, propylene glycol and poly(ethylene glycol). Alternatively, the compounds of the present invention may be freeze-dried together with excipients such as sugars (e.g. inositol, mannitol, glucose, mannose, maltose and sucrose) and amino acids (e.g. phenylalanine). Before intravenous injection, such freeze-dried compounds are dissolved in suitable solvents such as sterilized water, physiological saline, glucose solution, electrolyte solution and amino acid solution. The content of the compounds of the present invention in the immunosuppressive agent varies with the specific type of formulation, and it generally ranges from 0.1 to 100 wt%, preferably from 1 to 98 wt%. With injections, the content of the active ingredient generally ranges from 0.1 to 30 wt%, preferably from 1 to 10 wt%. For oral administration, the compounds of the present invention may be mixed with any of the solid or liquid carriers listed above, and used in the form of tablets, capsules, powders, granules, liquids or dry syrups. With capsules, tablets, granules and powders, the content of the active ingredient ranges generally from 5 to 100 wt%, preferably from 25 to 98 wt%.

The dosage of the immunosuppressant containing the compounds of the formula I as the active ingredient according to the present invention should be properly determined depending upon the age and body weight of the patient, as well as the severity and type of the disease. The effective dose generally ranges from 1 to 100 mg/kg per day for parenteral administration, and from 5 to 500 mg/kg per day for oral administration.

The compounds of the formula I are characterized by relatively low toxicity and small accumulation of toxicity upon continuous administration. The $LD_{50}$-values of compounds (1) to (11) for a single intraperitoneal administration to mice ar shown in Table 2.

Table 2

| Toxicity to mice | |
|---|---|
| Compound No. | $LD_{50}$ (mg/kg) |
| (1) | 25 ~ 50 |
| (2) | 12.5 ~ 25 |
| (3) | 12.5 ~ 25 |
| (4) | 12.5 ~ 25 |
| (5) | 12.5 ~ 25 |
| (6) | 25 ~ 50 |
| (7) | 25 ~ 50 |
| (8) | 25 ~ 50 |
| (9) | 25 ~ 50 |
| (10) | 25 ~ 50 |
| (11) | 25 ~ 50 |

The compounds of the formula I exert inhibitory effects on the function of lymphocytes of immunological importance. Tests by the method of Waithe et al. (Waithe et al., Handbook of Experimental Immunology, page 26.1, 1978), revealed that the compound of the formula I appreciably inhibited the T-lymphocyte blast genesis stimulated by concanavalin A (Con A) and the reaction of B-lymphocyte blast genesis stimulated by lipopolysaccharide (LPS). Therefore in vivo experiments have been conducted to examine the effects of the compounds of the formula I on T-lymphocytes. First, in accordance with the method of Lagrange et al. (Lagrange et al., Journal of Experimental Medicine, 139, 528-542, 1974), the compounds of the formula I were examined for their effects on delayed-type hypersensivity in mice that had been sensitized with sheep red blood cells. The delayed-type hypersensivity could be blocked by

administering the compound for three consecutive days following the sensitization with sheep red blood cells. Additionally, in accordance with the method of Jerne et al. (Jerne et al., Cell-bound Antibodies, pp. 109-122, 1963), the compounds of the formula I were checked for their effects on the plaque-forming ability of spleen cells from mice sensitized by antigenic sheep red blood cells. The compounds of the formula I also exhibited blocking effects in this test. It was therefore confirmed that the compounds of the formula I exhibited inhibitory action on the antibody production by B-lymphocytes.

The above data show the ability of the compounds of the formula I to inhibit the functions of B- and T-lymphocytes. The inhibited B- and T-lymphocytes respectively mean the suppression of humoral immunity and that of cell-mediated immunity. Therefore, the immunosuppressants according o the present invention containing the compounds of the formula I as the active ingredient are very effective in suppressing tissue rejection that accompanies the transplantation of an organ or skin probably because of an abnormally enhanced humoral or cellular immunity. The compounds can also be used for the treatment of diseases caused primarily by various forms of autoimmunity, as well as rheumatism and allergic diseases. The compounds of the formula I seem to have a different mechanism of action than the prior art immunosuppressants and have therefore no such serious side effects as causing disorder in hematopoietic organs as is caused by all immunosuppressants classified as cytotoxic substances, or the development of gastric ulcers and cataracts that often accompanies the administration of steroid hormones.

The following test results are provided for further illustrations of the pharmacologic effects of the compounds of the formula I.

Test Example 1

Inhibition of Delayed-Type Hypersensitivity in Mice Sensitized by Sheep Red Blood Cells

CDFl mice (8-wk-old, female) were sensitized by intravenous injection of 1 x $10^5$ sheep red blood cells. Four days later, the sensitized mice were subcutaneously injected in one footpad with 1 x $10^8$ sheep red blood cells so as to induce delayed-type hypersensitivity. The swelling of the footpad was measured at the 24th hour with a micrometer. Each of the test compounds was administered intraperitoneally in the amounts (mg/kg) indicated in Table 3 for a period of 3 days that started on the day following the initial sensitization. The degree of footpad swelling was indicated in relative values, with the value for a control given physiological saline instead of the test compounds being taken as 100%. The results are shown in Table 3, from which one can see the great ability of the compounds of the formula I to inhibit delayed type hypersensitivity.

Table 3 Inhibition of Delayed Type Hypersensitivity

| Compound No. | Footpad swelling (%) | | | | | | | |
|---|---|---|---|---|---|---|---|---|
| | 0.20 | 0.39 | 0.78 | 1.56 | 3.13 | 6.25 | 12.5 | 25 |
| (1) | 72 | | 87 | | 29 | | 15 | |
| (2) | 99 | | 49 | | 5.5 | | | |
| (3) | 55 | | 32 | | 11 | | 6.6 | |
| (4) | | 99 | 111 | | | 46 | | |
| (5) | 81 | | 117 | | 58 | | | |
| (6) | | | | | | 102 | | 33 |
| (7) | | | | | | 54 | | 42 |
| (8) | | | | | 50 | | 20 | |
| (9) | | | | | 45 | | 52 | |
| (10) | | | | | 37 | | 6.0 | |
| (11) | | | | | 40 | | 8.5 | |
| Spergualin | | | | | 106 | | 28 | |
| Control (physiological saline) | 100 | 100 | 100 | 100 | 100 | 100 | 100 | 100 |

Text Example 2

Inhibition of ConA induced reaction of T-lymphocyte blast genesis

Spleen cells from BALB/C mice were distributed among wells in a microplate so that each well contained 2 x $10^5$ cells/0.2 ml. Each of the selected test compounds was added to all wells but one, the latter being used as a control. ConA (5 $\mu$g/ml) was added to all the wells and the so prepared cell suspensions were cultivated in a 5% $CO_2$ incubator for 72 hours at 37 °C. Eight hours before the completion of the incubation, 1 $\mu$Ci of $^3$H-thymidine was added to each well and the uptake of the thymidine by the cultured cells was measured with a liquid scintillation counter to estimate the progress of reaction of T-lymphocyte blast genesis. The percentage inhibition of the genesis by each of the test compounds was calculated by: (1 - Bdpm/Adpm) x 100 wherein Adpm indicates the uptake count for the addition of ConA alone and Bdpm, the count for the addition of both ConA and the test compound. The results are shown in Table 4.

Table 4 Inhibition of ConA-induced reaction of T-lymphocyte blast genesis

| Test Compound No. | Inhibition, % 10 μg/ml | 100 μg/ml |
|---|---|---|
| (1) | 93 | 100 |
| (4) | 99 | 100 |
| (5) | 96 | 100 |
| (6) | 99 | 100 |
| (8) | 79 | 100 |
| (11) | 88 | 100 |
| Control (no compound added) | 0 | 0 |

As Table 4 shows, test compound Nos. 1, 4, 5, 6, 8 and 11 of the above-mentioned formula exhibited a strong ability to inhibit the reaction of T-lymphocyte blast genesis.

Test Example 3

Inhibition of LPS induced reaction of B-lymphocyte blast genesis

The uptake of $^3H$-thymidine by B-cell blasts was measured in accordance with the method used in Test Example 2, except that the inducer ConA was replaced by 100 μg/ml of LPS from E. coli. The percentage inhibition of blast genesis by selected test compounds was also determined by the same method as used in Text Example 2. The results are shown in Table 5, from which one can see that compound Nos. 1, 4, 5, 6 and 11 of the above-mentioned formula were highly effective in suppressing the LPS-induced genesis of B-lymphocyte blasts.

Table 5 Inhibition of LPS-induced genesis of B-lymphocyte blasts

| Test Compound No. | Inhibition, % 10 μg/ml | Inhibition, % 100 μg/ml |
|---|---|---|
| (1) | 98 | 100 |
| (4) | 100 | 100 |
| (5) | 88 | 100 |
| (6) | 96 | 100 |
| (11) | 98 | 100 |
| Control (no compound added) | 0 | 0 |

Test Example 4

Inhibition of Production of Antibodies against Sheep Red Blood Cells

CDFI mice (6-10 wk old, female) were immunized by intravenous injection of 1 x $10^8$ sheep red blood cells. The mice were intraperitoneally administered 12.5 mg/kg per day of selected test compounds for a period of 3 days starting from the day following the intravenous injection. Four days later, spleen cells were isolated from the mice and the number of plaque-forming cells was counted. The percentage inhibition of antibody production was calculated by (1 - B/A) x 100 wherein A is the count for a control group (given physiological saline) and B, for the treated group. Compound Nos. 1, 2, 4, 5 and 6 exhibited inhibitions of 98.5%, 95.9%, 89,4%, 89,4% and 92,9%, respectively.

As is clear from the foregoing description, the compounds of the formula I have a relatively low toxicity ($LD_{50}$ between 12.5 and 50 mg/kg) and exhibit good immunosuppressive actions such as the inhibition of delayed-type hypersensitivity, the inhibition of the functions of T- and B-lymphocytes, and the inhibition of the production of antibodies against sheep red blood cells. The compounds of the formula I seem to have a different mechanism of action from the prior art immunosuppressants, and because of this fact, formula I are used for the preparation of immunosuppressing agents according to the present invention having lower side effects.

The following Examples are provided for further illustrations of the present invention.

Example 1

Thirty parts by weight of a hydrochloride of compound No. 1 was mixed with purified water to make a total of 2,000 parts. The solution was passed through a Millipore filter of GS type for sterilization purposes. Two grams of the filtrate was put into 10-ml vials and freeze-dried to prepare injections each containing 30 mg of the hydrochloride of compound (1) per vial.

Example 2: Granules

An intimate mixture of 50 parts by weigth of a hydrochloride of compound (2), 600 parts of lactose, 330 parts of crystalline cellulose and 20 parts of hydroxypropyl cellulose was compacted with a Roller Compactor (R), and ground into particles which were sieved to provide granules of a size between 16 and 60 mesh.

Example 3: Tablets

A mixture of 30 parts by weight of a hydrochloride of compound (3), 120 parts of crystalline lactose, 147 parts of crystalline cellulose and 3 parts of magnesium stearate was processed with a V-type pelletizing machine to produce tablets each weighing 300 mg.

The following Reference Examples are given to show the methods of synthesizing the compounds of the formula I. In the Reference Examples, diZ, TAD, and GP represent dibenzyloxycarbonyl, triazadecane and guanidinophenyl, respectively.

Reference Example 1

N-[4-(3-aminopropyl)aminobutyl]-2-[4-(p-guanidinophenyl)butaneamido]-2-hydroxymethylethaneamide (compound No. 7)

(1) N-[4-(3-benzyloxycarbonylaminopropyl)benzyloxycarbonylaminobutyl]-2-[4-(p-guanidinophenyl)-butaneamido]-2-benzyloxymethylethaneamide

1.26 g (4.89 mmol) of brown crystalline 4-(4-GP)butyratehydrochloride was dissolved in 20 ml of dimethylformamide. To the ice-cooled solution, 0.68 g (5.87 mmol) of N-hydroxysuccinimide and 1.20 g (5.87 mmol) of N,N'-dicyclo-hexylcarbodiimide were added and the mixture was held overnight at room temperature. The precipitating N,N'-dicyclohexylurea was filtered off and the filtrate was immediately used in the subsequent reaction. 3.54 g (6.00 mmol) of a pale yellow oil of 10-(O-benzyl-L-ceryl)-1,5-diZ-1,5,10-TAD was dissolved in 30 ml of dimethylformamide. To the ice-cooled solution, 0.61 g (6.00 mmol) of triethylamine was added. After addition of the separately prepared dimethylformamide solution of the N-hydroxysuccinimide ester of 4-(4-GP)butyratehydrochloride, the mixture was left to stand overnight at room temperature. The reaction mixture was concentrated under vacuum and the only residue was dissolved in a mixture of 150 ml of ethyl acetate and 150 ml of chloroform. The solution was washed successively with 5% aqueous sodium carbonate, 0.5 N HCl and saturated aqueous NaCl. The organic layer was dried over anhydrous sodium sulfate and the desiccator was filtered off. Upon concentration of the filtrate under vacuum, a pale yellow oil of the end compound was obtained in an amount of 4.10 g (yield: quantitive).

TLC (chloroform : methanol : 17% aqueous ammonia = 6 : 1.5 : 0.25 v/v)

Rf. = 0.16.

The 4-(4-GP)butyratehydrochloride was synthesized by the following method.

1.60 g (8.93 mmol) of brown crystalline 4-(4-aminophenyl)butyric acid was dissolved in 40 ml of tetrahydrofuran. To the solution, 2.70 g (13.4 mmol) of a nitrate salt of 1-amidino-3,5-dimethylpyrazole and 2.19 g (17.0 mmol) of N,N-diisopropylethylamine were added, and the mixture was subjected to heating overnight under reflux. The precipitating crystal was recovered by filtration and washed successively with acetone, methanol and tetrahydrofuran. The dried brown crystal was suspended in 10 ml of distilled water and 1 N HCl was added until the crystal dissolved completely. Following concentration to dryness under vacuum, the residue was washed twice, each time with ether and acetone, thereby producing 1.54 g of a brown crystal (yield: 67.0%). m.p. 157 - 160 ° C.

(2) N-[4-(3-aminoproyl)aminobutyl]-2-[4-(p-guanidinophenyl)butaneamido]-2-hydroxymethylethaneamide

4.06 g (4.89 mmol) of a pale yellow oil of N-[4-(3-benzyloxycarbonylaminopropyl)-benzyloxycarbonylaminobutyl]-2-(7-guanidinophenyl)butaneamido]-2-benzyloxymethylethaneamide hydrochloride was dissolved in a mixture of methanol (50 ml) and acetic acid (30 ml). After addition of palladium black (0.4 g), the solution was heated to 55 ° C, at which temperature catalytic reduction was performed for 6 hours at atmospheric pressure. After completion of the reaction, the catalyst was filtered off, and the filtrate was concentrated under vacuum to obtain 2.80 g of an oily product. The oil was dissolved in 25 ml of 0.3 M NaCl in 60% (v/v) aqueous methanol, and the solution was passed through a column of 350 ml of CM-Sephadex (R) C-25 ($Na^+$) that had been equilibrated with the same solvent.

The column was eluted by the gradient elution between 2,000 ml of 0.3 M NaCl in 60% (v/v) aqueous methanol and 2,000 ml of 0.1 M NaCl in 60% (v/v) aqueous methanol. The fractions containing the end compound were collected and concentrated to dryness under vacuum. Methanol was added to the residue and the insoluble NaCl was filtered off. The resulting oil was purified by the following procedure.

In order to remove the small amount of residual NaCl, the oil was dissolved in 5 ml of methanol, and the solution was passed through a column filled with 100 ml of Sephadex (R) LH-20, followed by elution with

methanol, collection of the active fractions and concentration thereof under vacuum. In order to remove the small amount of the still remaining impurities, the oil was dissolved in 5 ml of distilled water, and the solution was passed through a column filled with 80 ml of HP-20(R) (product of Misubishi Chemical Industries Limited), followed by elution with distilled water, collection of the active fractions and concentration thereof under vacuum. The resulting oil was dissolved in 5 ml of distilled water and the insoluble matter was filtered off. The filtrate was freeze-dried to produce 1.17 g of the end compound (yield: 44.0%).
TLC (n-propanol : pyridine : water : acetic acid = 6 : 4 : 3 : 2 v/v)
Rf = 0.34

$$[\alpha]_D^{19.5} - 13.0°C\ (C = 1.17,\ H_2O)$$

Reference Example 2

N-[4-(3-aminopropyl)aminobutyl]-2-[4-(p-guanidinophenyl)butaneamido]-ethaneamide (compound No. 6)

(1) 10-(N,N'-phthalylglycyl)-1,5-diZ-1,5,10-TAD

12.4 g (30.0 mmol) of 1,5-diZ-1,5,10-TAD was dissolved in 200 ml of tetrahydrofuran. To the ice-cooled solution, 4.90 ml (35.0 mmol) of triethylamine and 10.6 g (35.0 mmol) of an ester of phthalylglycine and N-hydroxysuccinimide were added, and the mixture was left overnight at room temperature.

The reaction mixture was concentrated to dryness under vacuum and the residue was dissolved in 1200 ml of ethyl acetate. The ethyl acetate solution was washed successively with 5% aqueous sodium hydrogencarbonate, 0.5 N HCl and saturated aqueous NaCl. The ethyl acetate layer was dried on anhydrous sodium sulfate, and the desiccator was filtered off whereas the filtrate was concentrated under vacuum. To the resulting residue, ethyl acetate and ethyl ether were added, and the resulting crystal was recovered by filtration and dried to give 14.6 g of the end compound (yield: 81.0%). m.p. 102 - 104° C
TLC (chloroform : methanol : acetic acid = 95 : 5 : 3 v/v)
Rf = 0.4.

(2) 10-glycyl-1,5-diZ-1,5,10-TAD

To 14.4 g (24.0 mmol) of the 10-(N,N-phthalylglycyl)-1,5-diz-1,5,10-TAD, 370 ml of ethanol and 6.00 g (120 mmol) of hydrazine hydrate were added, and the mixture was refluxed for 2 hours. After completion of the reaction, the insoluble matter was filtered off and the filtrate was concentrated under vacuum. The resulting oil was dissolved in 300 ml of ethyl acetate and the solution was washed successively with 5% aqueous sodium hydrogencarbonate and distilled water. The ethyl acetate layer was dried on anhydrous sodium sulfate. After filtering off the desiccator, the filtrate was concentrated under vacuum to give 12.5 g of the end compound as an oil (yield: quantitative).
TLC (chloroform : methanol : acetic acid = 95 : 5 : 3 v/v)
Rf = 0.10

(3) N-[4-(3-benzyloxycarbonylaminopropyl)benzyloxycarbonylaminobutyl]-2-[4-(p-guanidinophenyl)-butaneamido]ethaneamide

1.56 g (6.05 mmol) of a brown crystalline 4-(4-GP)butyrate hydrochloride was dissolved in 20 ml of dimethylformamide. To the ice-cooled solution, 0.84 g (7.26 mmol) of N-hydroxysuccinimide and 1.50 g (7.26 mmol) of N,N'-dicyclohexylcarbodiimide were added, and the mixture was held at room temperature overnight. The precipitating N,N'-dicyclohexylurea was filtered off and the filtrate was immediately used in the subsequent reaction.

2.59 g (5.5 mmol) of a pale yellow oil of 10-glycyl-1,5-diZ-1,5,10-TAD was dissolved in 30 ml of dimethylformamide. To the ice-cooled solution, 0.61 g (6.05 mmol) of triethylamine was added. After addition of the separately prepared dimethyl formamide solution of the ester of 4-(4-GP)-butyratehydrochloride and N-hydroxysuccinimide, the mixture was held at room temperature overnight. The reaction mixture was concentrated under vacuum and the resulting oily residue was dissolved in a mixture

of ethyl acetate (200 ml) and ethanol (60 ml). The solution was washed successively with 5% phosphoric acid, 5% aqueous sodium carbonate and saturated aqueous NaCl. Any oil that came out of the solution during washing was dissolved by addition of a small amount of ethanol. The organic layer was dried on anhydrous sodium sulfate. After filtering off the desiccator, the filtrate was concentrated under vacuum to give 3.30 g of the end compound as a pale yellow oil (yield: 89.1%).
TLC (chloroform : methanol : 17% aqueous ammonia = 6 : 3.5 : 1 v/v).
Rf = 0.59.

(4) N-[4-(3-aminopropyl)aminobutyl]-2-[4-(p-guanidinophenyl)-butaneamido]-ethaneamide

3.30 g (4.90 mmol) of the pale yellow oil prepared in (3) was dissolved in 40 ml of acetic acid. To the solution, 0.3 g of palladium black was added and the mixture was heated to 50 °C, at which temperature catalytic reduction was conducted for 10 hours at atmospheric pressure. After completion of the reaction, the catalyst was filtered off and the filtrate was concentrated under vacuum to give 2.10 g of an oil.

The oil was dissolved in 10 ml of distilled water and the solution was passed through a column packed with 220 ml of CM-Sephadex(R) C-25 ($Na^+$). The column was then eluted by the gradient elution between 1,100 ml of distilled water and 1,100 ml of an aqueous solution of 1.0 M NaCl. The fractions containing the end compound were collected and concentrated to dryness under vacuum. Methanol was added to the dry product and the insoluble NaCl was filtered off.

Purification by the method used in Reference Example 1 gave 0.89 g of the end compound (yield: 35.3%).
TLC (n-propanol : pyridine : water : acetic acid = 6 : 4 : 3 : 2 v/v)
Rf = 0.26

## Reference Example 3

N-[4-(3-aminoproyl)aminobutyl]-2-[3-(p-guanidinomethylphenyl)propaneamido]-(S)-2-hydroxymethylethaneamide (compound No. 8)

(1) N-[4-(3-benzyloxycarbonylaminopropyl)benzyloxycarbonylaminobutyl]-2-[3-(p-guanidinomethylphenyl)-propaneamido]-(S)-2-benzyloxymethylethaneamide

One gram (4.52 mmol) of a pale yellow crystal of 3-(4-guanidinomethylphenyl)propionic acid was added in 4-5 small portions to 3 ml of ice-cooled thionyl chloride. Thereafter, the mixture was subjected to reaction for 15 minutes under cooling with ice. The reaction mixture was then concentrated to dryness under vacuum. Two grams (3.38 mmol) of 10-(O-benzyl-L-ceryl)-1,5-diZ-1,5,10-TAD was dissolved in 10 ml of dimethylformamide. To the ice-cooled solution, 0.92 g (9.04 mmol) of triethylamine was added. After addition of a solution in 4 ml of dimethylformamide of the separately prepared 3-(4-GP)priopionyl chloride hydrochloride, the mixture was subjected to reaction for 30 minutes under cooling with ice.

The reaction mixture was concentrated under vacuum and the oily residue was dissolved in a mixture of ethyl acetate (300 ml) and ethanol (50 ml). The solution was washed successively with 5% phosphoric acid, 5% aqueous sodium carbonate and saturated aqueous NaCl. Any oil that came out of the solution during the washing was dissolved by addition of a small amount of ethanol. The organic layer was dried on anhydrous sodium sulfate. After filtering off the desiccator, the filtrate was concentrated under vacuum to give 2.67 g of the end compound as a pale yellow oil (yield: quantitative).
TLC (chloroform : methanol : 17% aqueous ammonia = 6 : 1.5 : 0.25 v/v)
Rf = 0.27

The 3-(4-guanidinomethylphenyl)propionic acid used as the starting material was synthesized by the following procedure.

(a) methyl ester of 3-(4-aminomethylphenyl)propionic acid 4.30 g (22.97 mmol) of a white crystal of methyl 3-(4-cyanophenyl)propenate was dissolved in 350 ml of ammonia-saturated methanol. To the solution, 3 g of Raney nickel was added and 60 atmospheres of hydrogen was supplied for 2 hours at room temperature. After completion of the reaction, the catalyst was filtered off and the filtrate was concentrated under vacuum to give 4.02 g of an oil (yield: 90.54%).
TLC (chloroform : methanol = 10 : 1 v/v)
Rf = 0.16.

(6) 3-(4-guanidinophenyl)propionic acid

3.70 g (19.14 mmol) of the oily methyl ester of 3-(4-aminomethylphenyl)propionic acid was dissolved in 150 ml of tetrahydrofuran. To the solution were added 5.80 g (28.71 mmol) of 1-amidino-3,5-dimethylpyrazole nitrate and 4.70 g (36.37 mmol) of N,N-diisopropylethylamine, and the mixture was heated overnight under reflux. The reaction mixture was concentrated under vacuum to give an oil. This oil was reacted with 70 ml of 5% HCl for 3 hours under reflux. The reaction mixture was passed through a filter and the filtrate was ice-cooled. The pH of the filtrate was adjusted to 6.4 by addition of 10% aqueous NaOH. The so adjusted filtrate was stirred for 30 minutes under ice-cooling. The precipitating crystal was recovered by filtration and washed successively with distilled water and tetrahydrofuran. Upon drying, 2.85 g of a pale yellow crystal was obtained (yield: 67.4%). m.p. > 300 ° C.

(2) N-[4-(3-aminopropyl)aminobutyl)-2-[3-(p-guanidinomethylphenyl)propaneamido]-(S)-2-hydroxymethylethaneamide

2.60 g (3.27 mmol) of the pale yellow oil obtained in (1) was dissolved in a mixture of methanol (40 ml) and acetic acid (30 ml). After addition of palladium black (0.20 g), the solution was heated to 55 ° C, at which temperature catalytic reduction was conducted for 5 hours at one atmosphere. After completion of the reaction, the catalyst was filtered off and the filtrate was concentrated under vacuum to give 1.8 g of an oil. The oil was dissolved in 10 ml of distilled water, and the solution was passed through a column packed with 220 ml of CM-Sephadex$^{(R)}$ C-25 ($Na^+$), followed by elution by the gradient elution between 1,100 ml of distilled water and 1,100 ml of an aqueous solution of 1.2 M NaCl. The fractions containing the end compound were collected and concentrated to dryness under vacuum. Methanol was added to the dry product and the insoluble NaCl was filtered off.

By purifying the oil in accordance with the method used in Reference Example 2, the end compound was obtained in an amount of 0.84 g (yield: 47.2%).

Rf = 0.25

$$[\alpha]_D^{19.5} - 24.8°C \ (C = 1.0,\ H_2O)$$

Reference Example 4

N-[4-(3-aminopropyl)aminobutyl]-2-[5-(p-guanidinophenyl)pentaneamino]-(S)-2-hydroxymethylethaneamide (compound No. 9)

(1) N-[4-(3-benzyloxycarbonylaminopropyl)benzyloxycarbonylaminobutyl]-2-[5-(p-guanidinophenyl)-pentaneamido]-(S)-2-benzyloxymethylethaneamide

0.80 g (3.40 mmol) of a pale yellow crystal of 5-(4-GP)pentanoic acid and 1.80 g (3.05 mmol) of a pale yellow oil of 10-(O-benzyl-L-ceryl)-1,5-diZ-1,5,10-TAD were treated as in Reference Example 3 to produce 2.42 g of the end compound as a pale yellow oil (yield: quantitive).

TLC (chloroform : methanol : 17% aqeous ammonia = 6 : 1.5 : 0.25 v/v)

Rf = 0.38

The 5-(4-GP)pentanoic acid was synthesized by the following method.

7.42 g (35.80 mmol) of an oil of methyl 5-(4-aminophenyl)pentanoate was treated as in Reference Example 3, whereupon 3.72 g of the end compound was obtained as a pale yellow crystal (yield: 44.1%). m.p. 254 - 256 ° C.

(2) N-[4-(3-aminopropyl)aminobutyl]-2-[5-(p-guanidinophenyl)-pentaneamido]-(S)-2-hydroxymethylethaneamide

2.42 g (3.00 mmol) of the pale yellow oil prepared in (1) was treated as in Reference Example 3 to give 0.69 g of the end compound (yield: 41.1%).

TLC (chloroform : methanol : 17% aqueous ammonia = 4 : 4 : 2 v/v)

Rf = 0.50.

## Claims

1. Use of a compound of the formula I

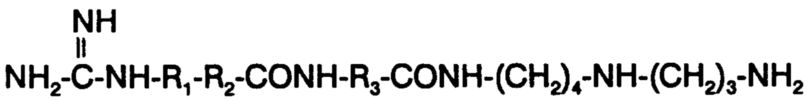 (I)

wherein $R_1$ is $-(CH_2)_4-$, $-(CH_2)_6-$

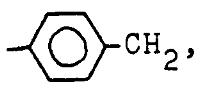, 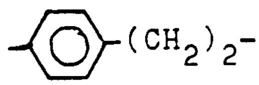

or

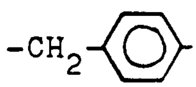 ;

$R_2$ is $-(CH_2)_2-$ or $-CH=CH-$;
and $R_3$ is

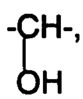 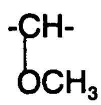

or $-CH_2-$
or pharmacologically acceptable salts thereof,
for the preparation of a medicament having immunosuppressive activity.

2. Use of a compound of the formula I according to claim 1 for the preparation of a medicament for suppressing tissue rejection or for treatment of diseases caused primarily by various forms of autoimmunity.

3. Use of a compound of the formula I according to claim 1 or 2 wherein $R_1$ is $-(CH_2)_4-$ or $-(CH_2)_6-$;
$R_2$ is $-(CH_2)_2-$ or $-CH=CH-$;
and $R_3$ is

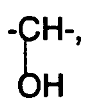 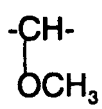

or $-CH_2-$
or pharmacologically acceptable salts thereof,
for the preparation of a medicament having immunosuppressive activity.

4. Use of a compound of the formula I according to one or more of the claims 1, 2 or 3 wherein $R_1$ is $-(CH_2)_4-$, $R_2$ is $-(CH_2)_2-$ and $R_3-$ is

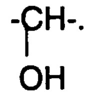

5. Use of a compound of the formula I according to one or more of the claims 1, 2 or 3 wherein $R_1$ is $-(CH_2)_6-$, $R_2$ is $-CH=CH-$ and $R_3$ is

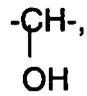

## Patentansprüche

1. Verwendung einer Verbindung der Formel I

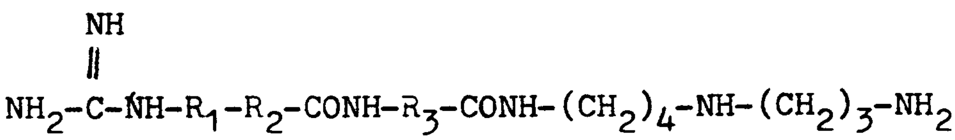
, (I)

in welcher $R_1$ $-(CH_2)_4-$, $-(CH_2)_6-$,

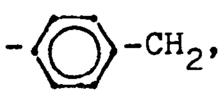, 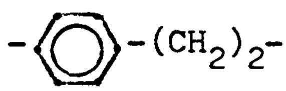

oder

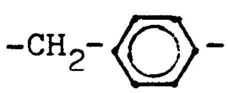

ist, $R_2$ $-(CH_2)_2-$ oder $-CH=CH-$ ist und $R_3$

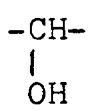, 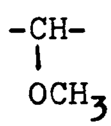

oder $-CH_2-$ bedeutet oder von pharmakologisch annehmbaren Salzen davon zur Herstellung eines Arzneimittels mit immunosuppressiver Wirkung.

2. Verwendung einer Verbindung der Formel I nach Anspruch 1 zur Herstellung eines Arzneimittels zur Unterdrückung von Gewebeabstossung oder zur Behandlung von Erkrankungen, die vorallem durch verschiedene Arten von Autoimmunität verursacht sind.

3. Verwendung einer Verbindung der Formel I nach Anspruch 1 oder 2, in welcher $R_1$ $-(CH_2)_4-$ oder

$-(CH_2)_6-$ ist, $R_2$ $-(CH_2)_2$ oder $-CH=CH-$ ist und $R_3$

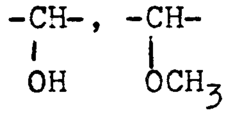

oder $-CH_2-$ bedeutet, oder von pharmakologisch annehmbaren Salzen davon zur Herstellung eines Arzneimittels mit immunosuppressiver Wirkung.

**4.** Verwendung einer Verbindung der Formel I nach einem oder mehreren der Ansprüche 1,2 oder 3, in welcher $R_1$ $-(CH_2)_4-$ist, $R_2$ $-(CH_2)_2-$ ist und $R_3$

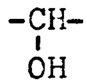

bedeutet.

**5.** Verwendung einer Verbindung der Formel I nach einem oder mehreren der Ansprüche 1,2 oder 3, in welcher $R_1$ $-(CH_2)_6-$ist,$R_2$ $-CH=CH-$ ist und $R_3$

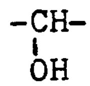

bedeutet.

## Revendications

**1.** Utilisation d'un composé de formule I

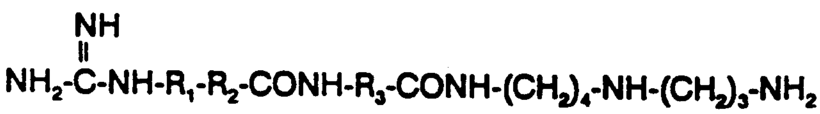 **(I)**

dans laquelle
$R_1$ est $-(CH_2)_4-$, $-(CH_2)_6-$,

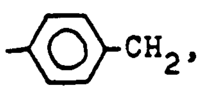 , 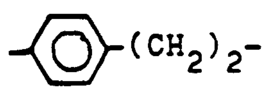

ou

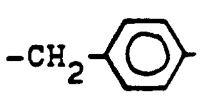 ,

$R_2$ est $-(CH_2)_2-$ ou $-CH=CH-$, et
$R_3$ est $-CH(OH)-$, $-CH(OCH_3)-$ ou $-CH_2-$,
ou leurs sels pharmacologiquement acceptables,
pour la préparation d'un médicament à activité immunodépressive.

2. Utilisation d'un composé de formule I selon la revendication 1, pour la préparation d'un médicament destiné à supprimer le rejet des tissus ou à traiter des maladies causées à l'origine par diverses formes d'auto-immunité.

3. Utilisation d'un composé de formule I selon la revendication 1 ou 2, dans lequel
$R_1$ est $-(CH_2)_4-$ ou $-(CH_2)_6-$,
$R_2$ est $-(CH_2)_2-$ ou $-CH=CH-$, et
$R_3$ est $-CH(OH)-$, $-CH(OCH_3)-$ ou $-CH_2-$,
ou leurs sels pharmacologiquement acceptables,
pour la préparation d'un médicament à activité immunodépressive.

4. Utilisation d'un composé de formule I selon une ou plusieurs des revendications 1, 2 ou 3, dans lequel $R_1$ est $-(CH_2)_4-$, $R_2$ est $-(CH_2)_2-$ et $R_3$ est $-CH(OH)-$.

5. Utilisation d'un composé de formule I selon une ou plusieurs des revendications 1, 2 ou 3, dans lequel $R_1$ est $-(CH_2)_6-$, $R_2$ est $-CH=CH-$ et $R_3$ est $-CH(OH)-$.